# EUROPEAN PATENT APPLICATION

(11) **EP 1 977 694 A1**
(43) Date of publication of application: **08.10.2008**
(21) Application number: 08006831.5
(22) Date of filing: 04.04.2008
(51) Int. Cl.: A61B 8/06

(54) **Ultrasound system and method of forming an ultrasound image**

(30) Priority: 06.04.2007 KR 20070034310
(71) Applicant: MEDISON CO., LTD., Kangwon-do 250-870 (KR)
(72) Inventor: Kye, Sang Bum, Gangnam-gu Seoul 135-280 (KR); Lee, Han Woo, Gangnam-gu Seoul 135-280 (KR)
(74) Representative: Schmid, Wolfgang

(57) **Abstract**

The present invention is directed to an ultrasound system and a method of forming an ultrasound image. The ultrasound system includes a signal processing unit, a processor, an input unit and a blood flow angle computation unit. The signal processing unit may form ultrasound image signals based on ultrasound echo signals reflected from a target object. The processor may form a B-mode image based on the ultrasound image signals. The input unit may allow a user to set a sample volume on the B-mode image. The blood flow angle computation unit may compute a blood flow angle in the sample volume. The signal processing unit may further compute a Doppler angle based on the blood flow angle and form Doppler signals based on the Doppler angle. Also, the processor may further form a Doppler spectrum image and Doppler sound based on the Doppler signals.

## Description

The present application claims priority from Korean Patent Application No. 10-2007-0034310 filed on April 06, 2007, the entire subject matter of which is incorporated herein by reference.

### BACKGROUND OF THE INVENTION

### [Technical Field]

The present invention generally relates to ultrasound systems, and more particularly to an ultrasound system and a method of forming an ultrasound image.

### [Background Art]

The ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

An ultrasound system generally uses a probe containing an array of piezoelectric elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce ultrasound data. The ultrasound data may include volume data obtained by using a 3-dimensional probe, etc.

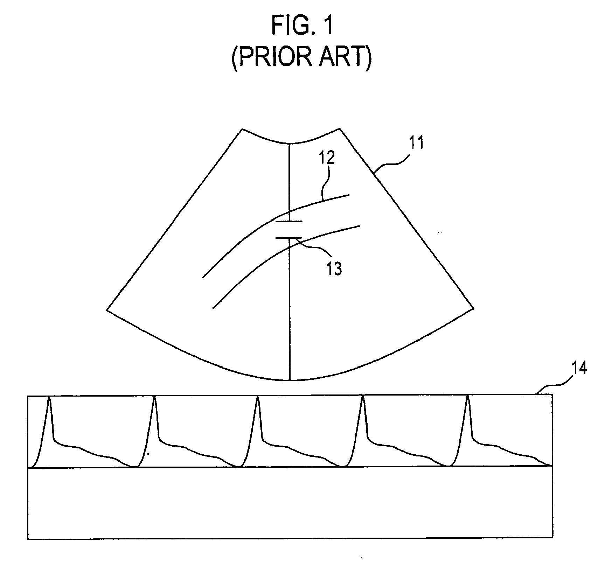

In the ultrasound system, the Doppler effect is used to measure the velocity of red blood cells flowing within a blood vessel or the velocity of a heart motion. FIG. 1 shows an example of displaying a B-mode image and a Doppler spectrum image at the same time. If a user sets a sample volume 13 on a blood vessel 12 in the B-mode image 11 by using a user input interface such as a track ball, then the ultrasound system repeatedly transmits/receives ultrasound signals to/from a region corresponding to the sample volume. The ultrasound system computes spectral Doppler components based on the reception signals and provides a Doppler spectrum image 14 or sound corresponding to the frequency or velocity based on the computed spectral Doppler components. The Doppler spectrum image 14 may indicate the motion direction and motion velocity of a moving object such as red blood cells or heart. In the Doppler spectrum image 14, a horizontal axis represents the time, while a vertical axis represents the velocity (or frequency).

After the sample volume is set on a blood vessel, a blood flow angle should be set by a user to compute the spectral Doppler components. However, inaccuracy may result from inputting the blood flow angle from the user through the input interface. Thus, the conventional ultrasound system may incorrectly compute the velocity of the blood flow. Also, a manual input operation of the blood flow angle by the user may lower the efficiency of the user's operation in a Doppler mode of the ultrasound system.

### BRIEF DESCRIPTION OF THE DRAWINGS

FIG. 1 is a schematic diagram showing an example of displaying a B-mode image and a Doppler spectrum image at the same time.

FIG. 2 is a block diagram showing an ultrasound system constructed in accordance with the present invention.

FIG. 3 is a schematic diagram showing edges of a blood vessel in a region of interest set to encompass a sample volume in accordance with the present invention.

FIG. 4 is a schematic diagram showing an example of a blood flow angle indicator in accordance with one embodiment of the present invention.

### DETAILED DESCRIPTION OF THE INVENTION

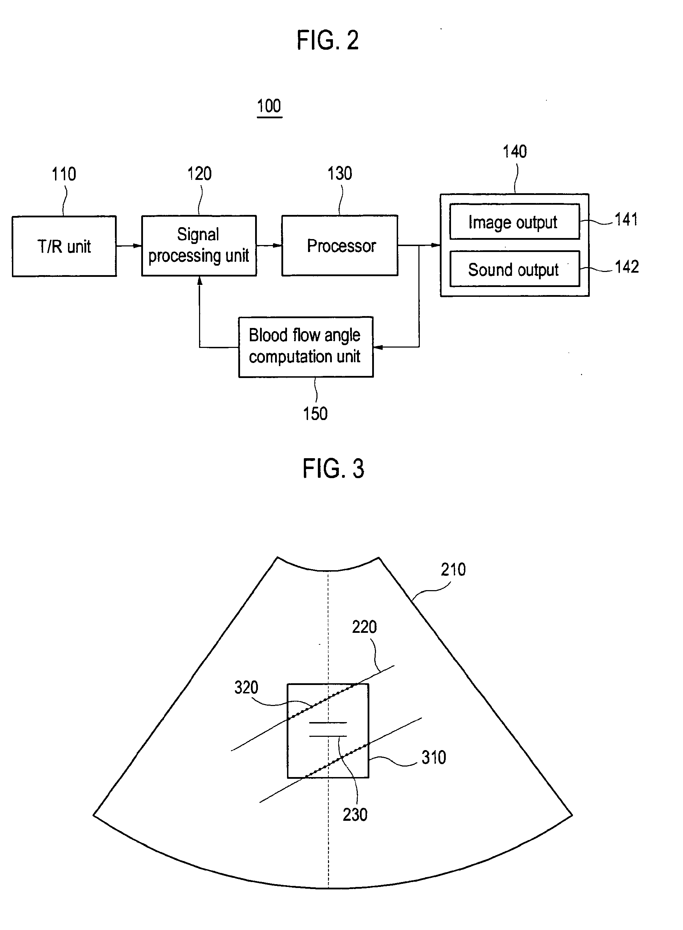

FIG. 2 is a block diagram showing an ultrasound system constructed in accordance with one embodiment of the present invention. As shown in FIG. 2, the ultrasound system 100 may include a transmission/reception (T/R) unit 110, a signal processing unit 120, a processor 130, an output unit 140 and a blood flow angle computing unit 150. The ultrasound system 100 may further include an input unit operable to allow a user to input a setup instruction for setting a sample volume. A size and position of the sample volume may be determined based on the setup instruction.

The T/R unit 110 may be operable to transmit ultrasound signals to a target object and receive ultrasound echo signals. The T/R unit 110 may include a probe containing a plurality of transducer elements, wherein each transducer element may reciprocally convert ultrasound signals and electrical signals. The T/R unit 110 may include a beam former for performing transmission focusing and reception focusing based on positions between the focal points and the transducer elements.

The signal processing unit 120 may be operable to perform signal processing upon reception-focused signals in the beam former to thereby acquire ultrasound image signals (e.g., B-mode image signals) or Doppler signals (e.g., Doppler spectrum image signals and Doppler sound signals) depending on the operation modes of the ultrasound system 100. In forming the Doppler signals, a Doppler angle representing an angle between a blood flow and a transmission beam may be additionally considered.

The processor 130 may be operable to form the B-mode image based on the ultrasound image signals. The processor 130 may set the sample volume on the B-mode image in response to the setup instruction inputted through the input unit. The processor 130 may form the Doppler spectrum image and the Doppler sound based on the Doppler signals acquired from the sample volume.

The output unit 140 may include an image output unit 141 and a sound output unit 142, as illustrated in FIG. 2. The image output unit 141 may be a monitor for displaying a B-mode image and a Doppler spectrum image. The B-mode image and the Doppler spectrum image may be displayed in various types. The sound output unit 142 may include a loud speaker for outputting the Doppler sound.

The blood flow angle computation unit 150 may be operable to set a region of interest (ROI) 310 to encompass the sample volume 230 on the B-mode image, as illustrated in FIG. 3. The blood flow angle computation unit 150 may be operable to detect edges 320 of a blood vessel 220 within the ROI 310. The edges 320 may be detected based on a brightness change between the pixels consisting of the B-mode image. The brightness change may be determined by using a differential operator. In accordance with one embodiment of the present invention, the edges may be detected by using an edge mask such as Sobel, Prewitt, Robert, Laplacian of Gaussian, Canny or the like. Also, differences of eigenvalues computed by using a structure tensor may be used to detect the edges. The blood flow angle computation unit 150 may include a noise reduction filter operable to remove noises from the B-mode image prior to detecting the edges.

The blood flow angle computation unit 150 may compute the slope of the vessels based on the detected edges to thereby compute the blood flow angle based on the computed slope. The slope may be the slope of a line obtained by connecting the edges. Information upon the computed blood flow angle may be transmitted to the signal processing unit 120. The signal processing unit 120 may be operable to compute a Doppler angle based on the blood flow angle to thereby form the Doppler signals.

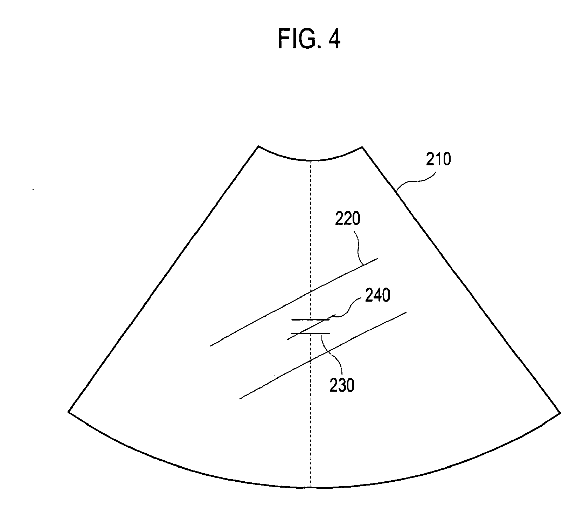

The blood flow angle computation unit 150 may control a blood flow indicator 240, which indicates the blood flow angle in the sample volume, as illustrated in FIG. 4. Although the blood flow angle is indicated by using the blood flowing indicator 240 in accordance with one embodiment of the present invention, the indication is not limited to the blood flowing indicator 240. Different types of indicators such as a text and the like may be used in accordance with another embodiment of the present invention.

As described above, the blood flow angle may be computed by using the slope of the blood vessel adjacent to the sample volume in accordance with the present invention. Thus, the velocity of the blood flow can be more accurately measured. Further, the inventive automatic setting feature of the blood flow angle may improve the efficiency of an operation in a Doppler mode of the ultrasound system.

In accordance with one embodiment of the present invention, there is provided an ultrasound system for forming an ultrasound signal, including: a signal processing unit operable to form ultrasound image signals based on ultrasound echo signals reflected from a target object; a processor operable to form a B-mode image based on the ultrasound image signals; an input unit operable to allow a user to set a sample volume on the B-mode image; and a blood flow angle computation unit operable to compute a blood flow angle in the sample volume, wherein the signal processing unit is further operable to compute a Doppler angle based on the blood flow angle and form Doppler signals based on the Doppler angle, and wherein the processor is further operable to form a Doppler spectrum image and Doppler sound based on the Doppler signals.

In accordance with another embodiment of the present invention, there is provided a method of forming an ultrasound image, including: a) forming first reception signals based on ultrasound echo signals reflected from a target object in response to first ultrasound transmission signals; b) forming a B-mode image based on the first reception signals; c) setting a sample volume on the B-mode image in response to a setup instruction inputted from a user; d) computing a blood flow angle in the sample volume; e) computing a Doppler angle based on the computed blood flow angle; f) forming second reception signals corresponding to the sample volume in response to second ultrasound transmission signals; g) forming Doppler signals based on the Doppler angle and the second reception signals; and i) forming a Doppler spectrum image based on the Doppler signals.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system for forming an ultrasound image, comprising:
a signal processing unit operable to form ultrasound image signals based on ultrasound echo signals reflected from a target object;
a processor operable to form a B-mode image based on the ultrasound image signals;
an input unit operable to allow a user to set a sample volume on the B-mode image; and
a blood flow angle computation unit operable to compute a blood flow angle in the sample volume;
wherein the signal processing unit is further operable to compute a Doppler angle based on the blood flow angle and form Doppler signals based on the Doppler angle, and wherein the processor is further operable to form a Doppler spectrum image and Doppler sound based on the Doppler signals.

2. The ultrasound system of Claim 1, wherein the blood flow angle computation unit is operable to set a region of interest (ROI) to encompass the sample volume, detect edges of a blood vessel in the ROI and compute the blood flow angle based on the detected edges.

3. The ultrasound system of Claim 2, wherein the angle computation unit is operable to use a slope of a line obtained by connecting the edges to compute the blood flow angle.

4. A method of forming an ultrasound image, comprising:
a) forming first reception signals based on ultrasound echo signals reflected from a target object in response to first ultrasound transmission signals;
b) forming a B-mode image based on the first reception signals;
c) setting a sample volume on the B-mode image in response to a setup instruction inputted from a user;
d) computing a blood flow angle in the sample volume;
e) computing a Doppler angle based on the computed blood flow angle;
f) forming second reception signals corresponding to the sample volume in response to second ultrasound transmission signals;
g) forming Doppler signals based on the Doppler angle and the second reception signals; and
i) forming a Doppler spectrum image based on the Doppler signals.

5. The method of Claim 4, wherein the step d) includes:
d1) setting a region of interest (ROI) to encompass the sample volume;
d2) detecting edges of a blood vessel in the ROI; and
d3) computing the blood flow angle based on detected edges.

6. The method of Claim 5, wherein the blood flow angle is computed by using a slope of a line obtained by connecting the edges.
